# EUROPEAN PATENT APPLICATION

(11) **EP 1 647 294 A1**
(43) Date of publication of application: **19.04.2006**
(21) Application number: 05107372.4
(22) Date of filing: 11.08.2005
(51) Int. Cl.: A61M 5/32

(54) **Syringe with retractable needle**

(30) Priority: 15.10.2004 US 966266
(71) Applicant: Intai Technology Corp., Taichung City 407 (TW)
(72) Inventor: Lou, Jin-Rong, DECEASED (CN)
(74) Representative: Jentschura, Rolf

(57) **Abstract**

The present invention provides a retractable safety syringe, which comprises a barrel (10), a sleeve (40), a connector (30), a plunger (20) and a standard needle. The barrel has the sleeve fitted at the front end. The connector is fitted in the sleeve and connected to the standard needle. The plunger is fitted in the rear lumen of the barrel for delivering fluid and retracting the connector with the standard needle into the barrel. The sleeve has threads on its inner surface. The connector is formed integrally by a cone-shaped cylinder and a tub-body, wherein the tub-body can be engaged with the plunger so as to retract the standard needle with the connector into the barrel by pulling the plunger. In the present invention, the barrel, the sleeve and the connector are separated.

## Description

### 1. Field of the Invention

The present invention relates to a safety syringe with a standard needle enable to retract into barrel, so that the injury can be prevented from the exposed standard needle. Particularly, it's specified a composition of safety syringe that can be compatible with standard needles, which comprise of the individual part of a barrel, a sleeve and a connector separately. Accordingly, the standard needle can be withdrawn back into barrel after use to reduce the possibility of needle stick accidentally.

### 2. Description of the Related Prior Arts

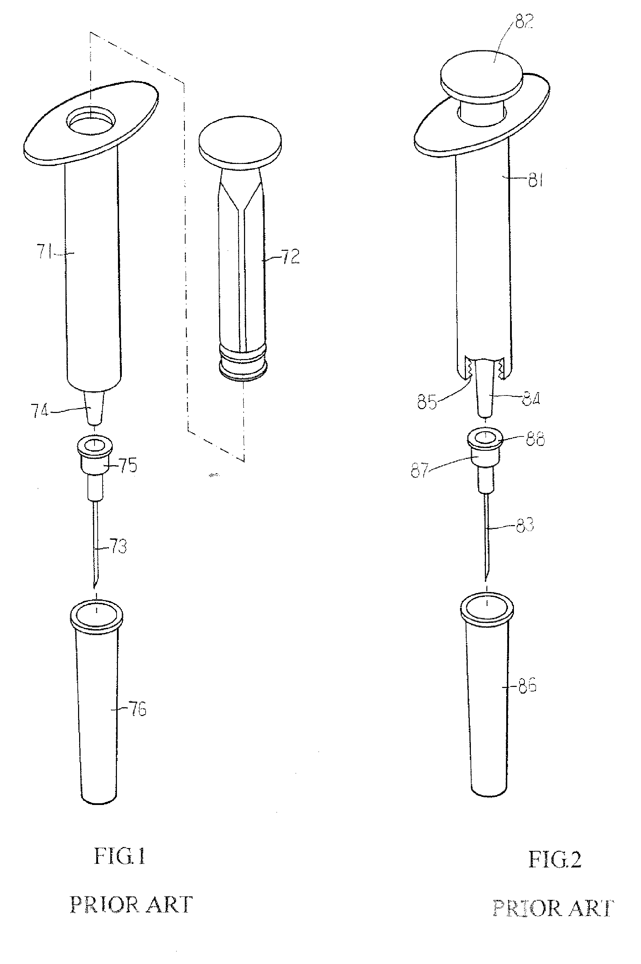

Fig. 1 shows a conventional syringe composed of a barrel (71), a plunger (72) and a standard needle (73). The barrel (71) protrude to form a conoid tube (74) at the front end. When operate the syringe injection, the needle hub (75) of standard needle (73) is pressed to seat on the cone-shaped cylinder (74) by hands. Then the plunger (72) is pulled to draw medicine fluid into barrel (71) therein. After an expected dosage is reached, an injection is given or a sheath (76) is recapped onto the standard needle (73) then loose and take off the standard needle (73) by hand from the cone-shaped cylinder and a new standard needle (73) is replaced. The medicine fluid is delivered through the barrel, the cone-shaped cylinder, the hub (75) of standard needle (73), and the cannula to physical body when pressing the plunger. As the standard needle (73) of such syringe is pressed to seat on the cone-shaped cylinder by hand, unexpected disconnection of the standard needle (73) may result in injury due to careless operation.

To prevent the previous hidden dangerous, another conventional syringe named Luer lock syringe have been developed as shown in Fig. 2 is provided. The syringe is composed of a barrel (81), a plunger (82) and a standard needle (83). On the front end of barrel a cone-shaped cylinder (84) and a thread (85) is integrally formed on its lumen. Before syringe injectionturn, turn the standard needle (83) clockwise then the hub flange of standard needle (83) can be pushed forward by thread to connect on the cone-shaped cylinder securely. By means of the thread withstanding to the hub (85) flange of standard needle (83), The unexpected disconnection of the standard needle (83) can be prevented. When using this syringe, a medicine fluid is sucked into barrel by the plunger pulling. After an expected dosage is reached, an injection is given or a sheath (86) is recapped and the standard needle (83) is turned counterclockwise to be loosened and taken off.

As the conventional syringe, that the cone-shaped cylinder and the thread are formed integrally with barrel, therefore the needle could not be retracted backward into barrel for needle stick protection. To solve this problem, a retractable safety syringe is provided, in which a barrel is attached with a special purpose needle (73) at its front end. When the plunger is pushed forward to the full stop of travel, an engagement will be happened between the plunger and the hub of special purpose needle (73). Accordingly, the special purpose needle can be retracted into barrel by pulling the plunger backward for needle stick protection. Then an injury due to an exposed needle can be avoided. However, the special purpose needle of such safety syringe cannot be replaced to a new needle to cause the possibility of needle pollution after drawing fluid from bottle or venous system.

Another disadvantage of the above retractable syringe is that the syringe can't be compatible with standard needle (73) so that a special purpose needle should be applied to this syringe. Therefore, it is effective to obstruct the wild application of safety syringe to cause the dangerous of needle stick could be happened on syringe operators.

### SUMMARY OF THE INVENTION

A primary object of the present invention is to provide a retractable safety syringe, in which a standard needle can be retracted into barrel, by mean of the composition of a barrel, a sleeve, a connector and a standard needle individually.

Another object of the present invention is to provide a retractable safety syringe, in which medicine fluid is contained in the syringe with leakage-proof during injection operation is performed due to the construction of a ring seal is melted with a sleeve.

A further object of the present invention is to provide a retractable safety syringe, in which the sleeve and the connector can be pre-assembled and then inserted the whole pre-assembly into the barrel from a front end thereof, and therefore assembly automation of the syringe is more simple and effective.

Accordingly, the barrel of the present invention is designed as a hollow tube. The sleeve is received in the front end of the lumen of barrel and the connector is received in the lumen of sleeve. The barrel is formed a protruding ring at a front end of lumen thereof, so that the connector can be withstood at the side of protruding ring. A cone-shaped cylinder of the connector is extruded to the front of the barrel for connecting with the standard needle. A plunger is installed in the barrel for sucking medicine fluid by pulling and pushing the plunger back and forth. When the plunger is pushed to the full stop of travel, the engagement will be happened on the connector and plunger. Therefore, the standard needle is connected with the connector and retracted back to barrel by pulling the plunger.

The sleeve with its tube-like body is tightly received in the front portion of barrel. The lumen of the sleeve has twin-twisted threads, and a rear end thereof is melted with a ring seal. As the outer surface of the sleeve is tightly fitted in the barrel, so that medicine fluid in the syringe could not be leakage from an interface of barrel and sleeve thereof.

The connector is integrally composed of a cone-shaped cylinder and a tub-body. The connector is fitted in the sleeve and connected with the standard needle on a cone-shaped cylinder. The cone-shaped cylinder of the connector is a hollow cylinder with increasing diameter from its outer end of cylinder to its bottom where integrated with the tub-body. By arrangement of the tub-body with one end withstanding at the side of protruding ring of barrel, and another end fitting in the lumen of sleeve then the connector can be fixfirmly fixed in expected position. Moreover, a ring seal is interferingly fitted surrounding the tub-body, so that leakage is prevented from an interface of the tub-body and the sleeve.

Further benefits and advantages of the present invention will become apparent after a careful reading of the detailed description with appropriate reference to the accompanying drawings.

### BRIEF DESCRIPTION OF THE DRAWINGS

Fig. 1 shows a conventional syringe;
Fig. 2 shows another conventional syringe;
Fig. 3 is an exploded perspective view of the first embodiment in accordance with the present invention;
Fig. 4 shows an axial cross section of the first embodiment in accordance with the present invention;
Fig. 5 shows a cross section view of Fig. 4 along 2-2;
Fig. 6 shows an axial cross section of the first embodiment in use in accordance with the present invention;
Fig. 7 shows an axial cross section of the second embodiment in accordance with the present invention.

### DETAILED DESCRIPTION OF THE INVENTION

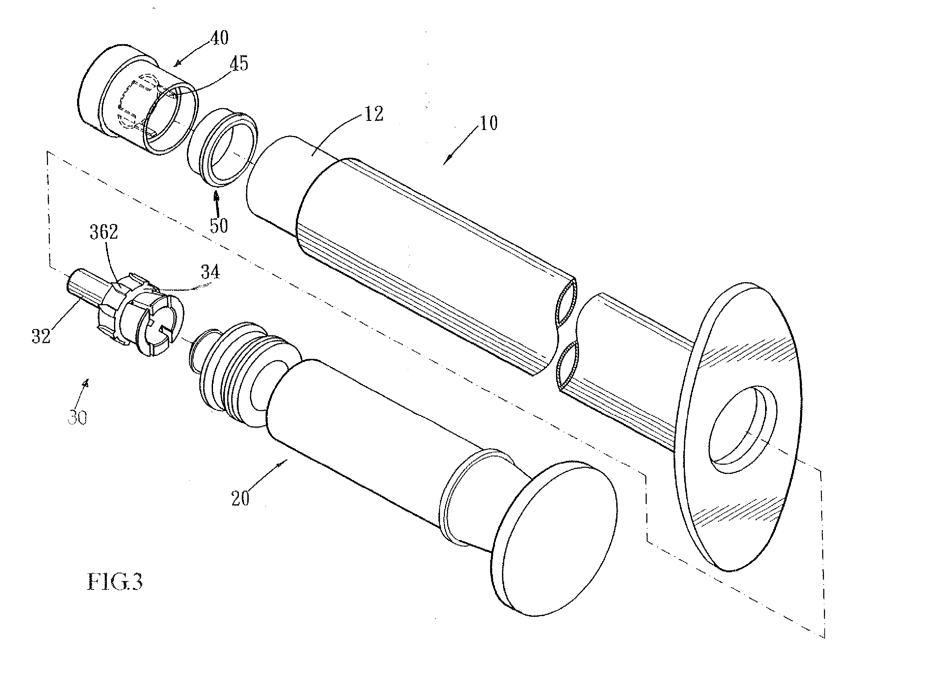

Referring to Figs. 3, 4 and 6, a retractable safety syringe of the present invention primarily comprises a barrel (10), a sleeve (40), a connector (30), a plunger (20) and a standard needle (90). The barrel (10) is designed as a hollow tube with an inwardly extended ring (13) at a front portion thereof, so that the barrel (10) is defined as a front lumen (12) and a rear lumen (14).

The sleeve (40) is a hollow tube with interference fitting into the front lumen (12) of the barrel (10). A ring seal (50) is melted to integrate a rear portion of the sleeve (40). The ring seal (50) is a thin wall cylinder made from resilient material, such as rubber, and also in interference fitting with the front lumen (12) of the barrel (10). Accordingly, medicine fluid in the syringe could not leak out from an interface of the front lumen (12) and the sleeve (40).

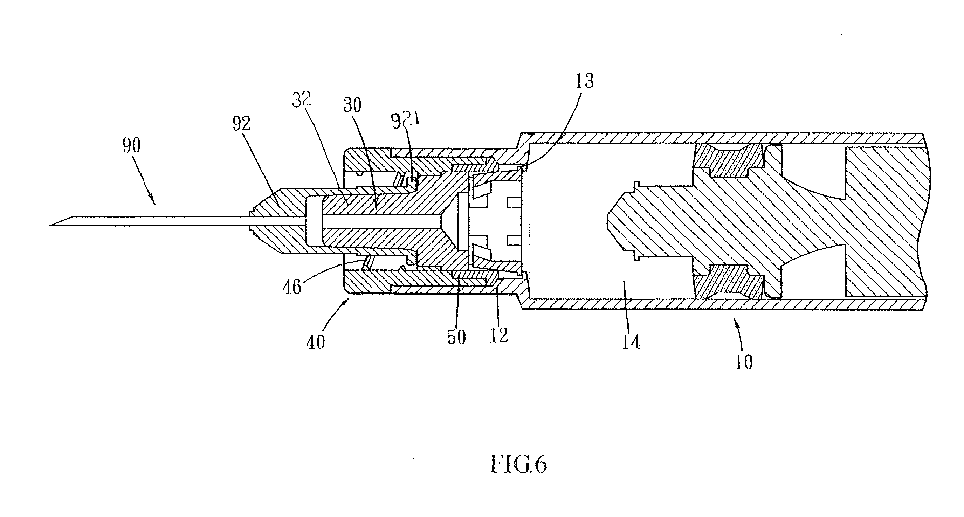

The sleeve (40) has twin-twisted threads (46) in its lumen thereof. Each thread (46) are formed in one end of sleeve and separate in a phase angle of 180 degree for each other, and the effective length of twin-twisted thread is equal or less to the half length of a full pitch threadwhen. Therefore, a hub flange (921) of standard needle (92) will be pushed to move inward by threads when standard needle is turned in clockwise, as shown in Fig. 6. Thus the hub (75) of standard needle (90) can be connected onto cone-shaped cylinder (32) of connector (30).

The connector (30) is integrally formed a cone-shaped cylinder (32) and a tub-body (34). The tub-body (34) fitted in the ring seal (50) of sleeve (40) with one end, and another end of the tub-body (34) is seat on the side of the protruding ring (13) of barrel (10). Therefore the connector (30) is firmly fixed in the front lumen (14) of barrel (10). The cone-shaped cylinder (32) of the connector is a hollow tube with increasing diameter from its outer end to its bottom that looks like a cone-shaped cylinder, where integrated with the tub-body. The tub-body (34) is fitted in the ring seal (50) of the sleeve (40) with a slight interference, and therefore medicine fluid in the syringe could not be leakage from an interface of the sleeve (40) and the connector (30).

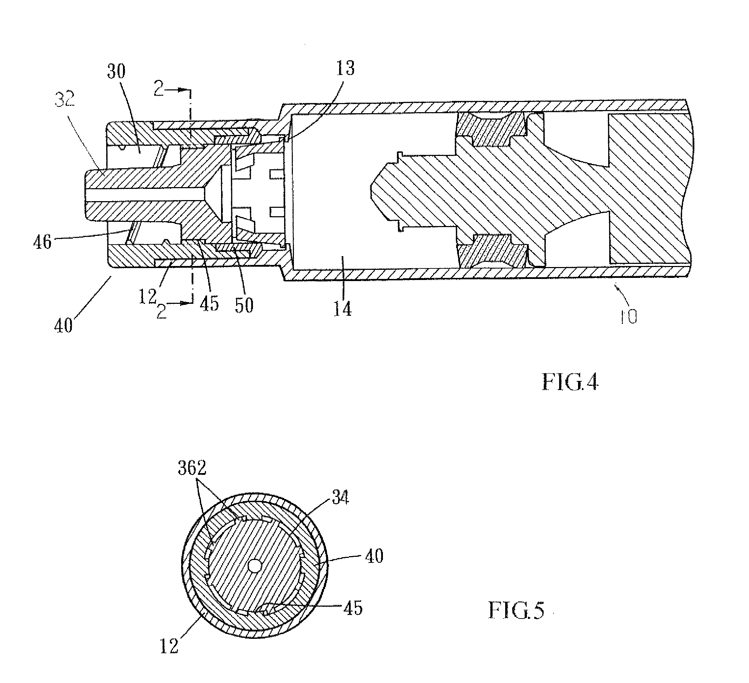

Fig. 5 shows a cross section view of Fig. 4 based on the line of 2-2, in which the tub-body (34) comprises of a plurality of splines (362) surrounding an outer surface thereof. Fig. 3 shows the feature of crowns (45) that formed on an inner surface of the sleeve (40). When the connector (30) is fitted in sleeve (40), the splines (362) and crowns (45) will be engaged together so that the connector can be prevented slipping around from engagement. Fig. 3 also shows arrangement of the splines (362) and crowns (45) respectively on the connector (30) and the sleeve (40).

In this embodiment, the barrel (10), the sleeve (40) and the connector (30) can be separated. When using the syringe, the standard needle (92) is screwed clockwise and the flange (921) of the standard needle (92) is pushed in one way by the threads (46) to make standard needle hub (75) firmly seating on the cone-shaped cylinder (32) of connector (30) as shown in Fig. 6. By means of construction of the thread (46) and the flange (921), an unexpected disconnection of the standard needle (90) also can be avoided. When the plunger (20) is pulled back and complete sucking medicine fluid into the barrel (10), an injection is given or recapthes the standard needle and turns the standard needle counterclockwise. Then another standard needle can be forreplaced for physical injection. When the plunger (20) is pressed to a full stop of travel, a front end of the plunger (20) will engage with the tub-body (34) of the connector (30). Accordingly, the standard needle (90) accompanied with the connector (30) can be retracted into the barrel (10) by pulling the plunger (20) backward. Mechanism for engaging the plunger (20) with the tub-body (34) is known technique by people in this art, and can be provided with any proper structures.

To assembly the syringe of this embodiment, the connector (30) can be fitted in the sleeve (40) first and then inserted the sleeve (40) together with connector (30) into the front lumen (12) of the barrel (10) from its front end. In other words, the connector (30) in the present invention is very easy to assembly.

Additionally, when the flange (921) of the standard needle (92) is pushed and moved to the close end of thread (46), the cone-shaped cylinder (32) also can be tightly theconnected with the standard needle (92) hub (75) with its cone-shaped cylinder. Medicine fluid for injection then can be sucked into the barrel by pulling the plunger. After a predetermined dosage of the medicine fluid is filled in the barrel, the standard needle is recapped with a sheath and turned counterclockwise for dismantling. The same procedures can be repeated to replace a new standard needle.

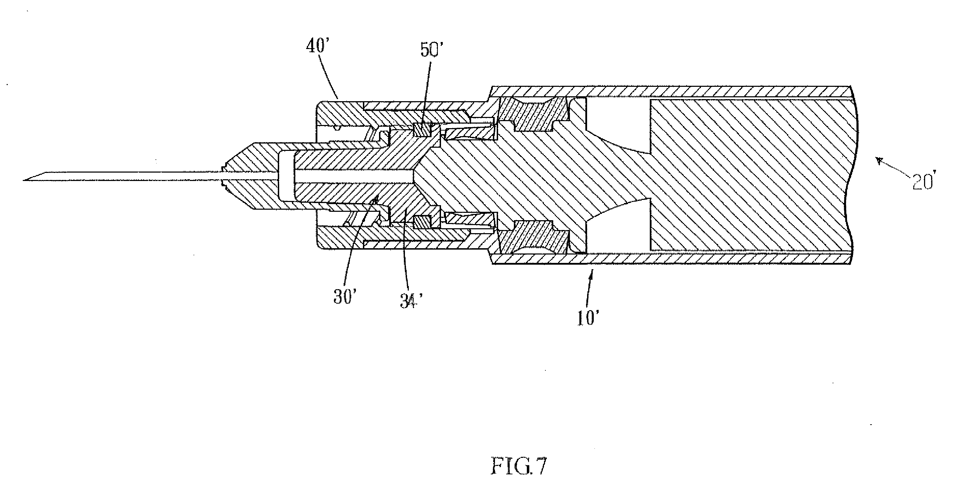

A second embodiment of the present invention is a modification of the first one. Fig. 7 shows a structure of the second embodiment, which primarily comprises a barrel (10'), a plunger (20'), a connector (30'), a sleeve (40') and a ring seal (50'). In this embodiment, the ring seal (50') is provided to closely surround a tub-body (34') of the connector (30'), and leaking of fluid can be prevented.

Although the present invention has been explained in relation to its preferred embodiments as mentioned above, it is to be understood that many other possible modifications and variations can be made without departing from the scope of the present invention. It is, therefore, contemplated that the appended claim or claims will cover such modifications and variations that fall within the true scope of the invention.

## Claims

1. A retractable safety syringe, comprising a barrel, a sleeve, a connector, a plunger and a standard needle; wherein the barrel is hollow tube and defines a front lumen and a rear lumen, the sleeve is fitted in the front lumen, the connector is fitted in the sleeve, and the plunger is fitted in the rear lumen for delivering andmedicine fluid and with drawing the connector with the standard needle into the barrel;
the sleeve is a tube with twin-twisted threads on its hole surface thereof, so that a flange of the standard needle can be guided by the threads to move inward to fit with cone-shaped cylinder of the connector;
the connector is integrally formed by a cone-shaped cylinder and a tub-body, wherein the tub-body can be engaged with the plunger to retract the standard needle into the barrel; the cone-shaped cylinder is a hollow tube which integrate with the tub-body to connect for the standard needle on the other end.

2. The retractable safety syringe as claimed in claim 1, wherein the sleeve, the connector and the barrel are separated each other.

3. The retractable safety syringe as claimed in claim 1, wherein the twin-twisted threads with the effective thread lengths is equal or less to the half length of a full pitch thread.

4. The retractable safety syringe as claimed in claim 1, which further comprises a ring seal to dispose interference fit at the interface of the sleeve fitting with the connector, and the barrel fitting with sleeve for leakage prevention.

5. The retractable safety syringe as claimed in claim 1, wherein the ring seal is a thin wall cylinder made from a resilient material and integrated with the sleeve by thermal melting.

6. The retractable safety syringe as claimed in claim 1, wherein the connector comprises a plurality of splines surrounding an outer surface thereof, so that the connector is prevented circumference slipping by the engagement of the spline and crowns formed on an inner surface of the sleeve.
